# EUROPÄISCHE PATENTSCHRIFT

(11) **EP 2 916 709 B1**
(45) Veröffentlichungstag und Bekanntmachung des Hinweises auf die Patenterteilung: **01.04.2020**
(21) Anmeldenummer: 13766507.1
(22) Anmeldetag: 20.09.2013
(51) Int. Cl.: A61B 1/00, G01M 3/26

(54) **ENDOSKOP UND DRUCKTESTVERFAHREN FÜR EIN ENDOSKOP**
ENDOSCOPE, AND PRESSURE-TESTING METHOD FOR AN ENDOSCOPE
ENDOSCOPE ET PROCÉDÉ D'ESSAI DE PRESSION POUR UN ENDOSCOPE

(30) Priorität: 08.11.2012 DE 102012220372
(43) Veröffentlichungstag der Anmeldung: 16.09.2015
(73) Patentinhaber: Digital Endoscopy GmbH, 86316 Friedberg (DE)
(72) Erfinder: PAUKER, Friedrich, 86420 Diedorf (DE)
(74) Vertreter: TBK
(86) Internationale Anmeldenummer: PCT/EP2013/069537
(87) Internationale Veröffentlichungsnummer: WO 2014/072110

(56) Entgegenhaltungen:
- EP-A2- 1 779 769
- DE-U1- 20 217 210

## Beschreibung

Die vorliegende Erfindung bezieht sich auf ein Endoskop, das für ein Drucktestverfahren geeignet ist, und auf ein Drucktestverfahren für ein Endoskop.

Endoskope sind zu einem wichtigen Hilfsmittel in der Medizin geworden, um Diagnosen und Eingriffe im Inneren des Körpers vorzunehmen.

Nach einem medizinischen Einsatz und der danach erfolgten Reinigung müssen Endoskope einem Druckdichtheitstest unterzogen werden. Dieser Druckdichtheitstest erfolgt über ein am Endoskop zu diesem Zweck angebrachtes Ventil und z.B. einer kleinen Handbalgpumpe. Das Endoskop wird mit einem vorbestimmten Druck (z.B. ein paar 100 mbar) unter Druck gesetzt, dabei wird ein an der Handpumpe angebrachtes Manometer beobachtet. Fällt innerhalb einer vorbestimmten Zeit (z.B. ca. 1 Minute) der Druck nicht ab, so kann das Instrument als dicht betrachtet werden.

Beim Prüfen der Druckdichtheit kann auf das zu diesem Zweck am Endoskop angebrachtes Ventil (ein Prüf- und Belüftungsventil) eine Schutzkappe aufgesetzt werden.

Ein solches herkömmliches Endoskop und ein solches herkömmliches Drucktestverfahren sind beispielsweise in der DE 202 17 210 U1 offenbart. Dabei sind an einem Versorgungsstecker eines Endoskops neben einem dem Aufsetzen einer Wasserschutzkappe dienenden Verbindungsansatz verschiedenartige Anschlüsse angebaut, die der Ausführung verschiedener Gerätefunktionen dienen. Mit der Wasserschutzkappe ist ein Prüf- und Belüftungsventil verschraubt, das der Durchführung eines Drucktests dient. Im Drucktest wird ein Dichtigkeitstester auf das Prüf- und Belüftungsventil aufgebracht, der aus einer Handpumpe mit Manometer, einem Verbindungsschlauch und einem Adapter zum Ansetzen an das Prüf- und Belüftungsventil besteht.

Wird durch den am Prüf- und Belüftungsventil durchgeführten Test ein Druckabfall festgestellt, so kann in einem Wasserbad geprüft werden, wo Luftperlen aufsteigen und somit das Leck verortet werden. Dieser Vorgang nimmt viel Zeit in Anspruch und bedeutet, dass über eine Öffnung das Innenleben des Endoskops mit der Umgebung verbunden werden kann. Dies birgt die Gefahr, dass Keime aus der Luft ins Innere des Endoskops gelangen können.

### DURCH DIE ERFINDUNG ZU LÖSENDE AUFGABE

Aufgabe der vorliegenden Erfindung ist es, ein verbessertes Endoskop und ein verbessertes Drucktestverfahren zu schaffen.

### LÖSUNG DER AUFGABE

In Hinblick auf das Endoskop ist diese Aufgabe erfindungsgemäß durch ein Endoskop mit den Merkmalen von Anspruch 1 gelöst. In Hinblick auf das Drucktestverfahren ist diese Aufgabe erfindungsgemäß durch ein Drucktestverfahren mit den Merkmalen von Anspruch 9 gelöst. Vorteilhafte Weiterbildungen sind Gegenstand der abhängigen Ansprüche.

Die Erfindung betrifft somit ein Endoskop mit einem Endoskopgriff und einem Endoskopschaft. Im Endoskopinnenraum sind ein Heizelement, ein Temperatursensor und ein Drucksensor angeordnet. Beim Druckdichtheitstest kann das Heizelement die im Endoskopinnenraum befindliche Luft erwärmen. Zugleich steigt der Druck im Endoskopinnenraum. Gemäß der thermischen Zustandsgleichung steigt dabei der Druck proportional zur Temperatur an. Steigt die Temperatur und der Druck bleibt konstant oder nimmt er nur geringfügig zu, kann gefolgert werden, dass das Endoskop undicht ist. Somit wird durch das neue Endoskop ein schneller und einfacher Druckdichtheitstest ausführbar, der zuverlässige Testergebnisse liefert. Da kein externes Medium in den Endoskopinnenraum eingepumpt werden muß, wird die Gefahr der Keimeinbringung vermieden.

Der Endoskopgriff und der Endoskopschaft können einen gemeinsamen Endoskopinnenraum ausbilden. Dabei können Endoskopgriff und Endoskopschaft den gemeinsamen Endoskopinnenraum derart ausbilden, dass dieser gegenüber der Umgebung abgedichtet ist.

Der Endoskopgriff und der Endoskopschaft können voneinander separierbar sind und jeweils einen eigenen Innenraum ausbilden. Der jeweilige Innenraum kann gegenüber der Umgebung abgedichtet sein.

Das Heizelement kann im Endoskopgriff angeordnet sein. Alternativ kann das Heizelement im Endoskopschaft angeordnet sein.

Das Heizelement kann durch zumindest einen elektrischen Verbraucher gebildet sein, der im Endoskop vorgesehen ist und bei Endoskopbetätigung involviert ist. In modernen Endoskopen befinden sich in der Regel Schaltkreise, welche eine Verlustleistung erzeugen, die wiederum in Form von Wärme an den Innenraum des Endoskops abgegeben wird. Somit ist es in einer Ausführungsform der Erfindung nicht notwendig, ein gesondertes Heizelement im Innenraum unterzubringen. Vielmehr reicht es, die elektronischen Schaltkreise in Betrieb zu nehmen und mit der damit verbundenen Erhöhung der Temperatur den Verlauf der Druckerhöhung zu verfolgen.

Der zumindest eine elektrische Verbraucher kann eine elektronische Auswerteschaltung oder eine Beleuchtungs-LED sein. Ein solcher elektrischer Verbraucher ist ohnehin im Endoskop vorgesehen. Er erhält lediglich die zusätzliche Aufgabe, bei der Dichtheitsprüfung als Heizelement zu dienen.

Alternativ kann der zumindest eine elektrische Verbraucher lediglich bei einer Dichtheitsprüfung aktivierbar sein. Der zumindest eine elektrische Verbraucher kann ein separates Heizelement sein, das bei Endoskopbetätigung nicht involviert ist. Für den Druckdichtheitstest kann somit ein spezifisches Heizelement vorgesehen werden, welches exakt für die technischen Gegebenheiten des Druckdichtheitstests konzipiert sein kann. Somit kann das Heizelement so ausgelegt sein, dass es genau die für den Druckdichtheitstest vorgesehene Wärmemenge abgeben kann. Somit kann vermieden werden, dass das Heizelement im Hinblick auf die Leistung oder Größe überdimensioniert wird.

Das erfindungsgemäße Drucktestverfahren für ein Endoskop weist die folgenden Schritte auf:
Aufwärmen des Innenraums des Endoskops,
Erfassen des Drucks während des Aufwärmens des Innenraums des Endoskops,
Erfassen der Temperatur während des Aufwärmens des Innenraums des Endoskops und
Vergleichen des erfassten Drucks und der erfassten Temperatur.

Beim Aufwärmen des Innenraums des Endoskops steigt die Temperatur der im Endoskopinnenraum befindlichen Luft an. Unter der Vorraussetzung, dass der Endoskopinnenraum gegenüber der Umgebung abgedichtet ist, steigt dabei gemäß der thermischen Zustandsgleichung der Druck proportional zur Temperatur an. Sollte die Temperatur zunehmen, der Druck aber konstant bleiben oder der Druck sich nur geringfügig erhöhen, kann gefolgert werden, dass das Endoskop undicht ist. Somit wird ein schnelles und einfaches Drucktestverfahren ausführbar, welches zuverlässige Testergebnisse liefert. Da kein externes Medium in den Endoskopinnenraum eingepumpt werden muß, wird ausserdem die Gefahr der Keimeinbringung vermieden.

Das Aufwärmen des Innenraums des Endoskops kann durch Aktivieren eines elektrischen Verbrauchers ausgeführt werden, der im Endoskop vorgesehen ist und bei Endoskopbetätigung involviert ist.

Alternativ kann das Aufwärmen des Innenraums des Endoskops durch Aktivieren eines separaten Heizelements ausgeführt werden, das bei Endoskopbetätigung nicht involviert ist.

Die vorstehend erläuterten Aspekte der vorliegenden Erfindung können geeignet kombiniert werden.

Nachstehend ist die vorliegende Erfindung detaillierter anhand von beigefügten Zeichnungen beschrieben.

### KURZBESCHREIBUNG DER ZEICHNUNGEN

Fig. 1 zeigt ein erstes Ausführungsbeispiel der vorliegenden Erfindung in einer schematischen aufgeschnittenen Ansicht.
Fig. 2 zeigt eine graphische Darstellung des Verlaufs der Temperatur und des Drucks in einem erfindungsgemäßen Drucktestverfahren, bei dem keine Undichtheit festgestellt wird.
Fig. 3 zeigt eine graphische Darstellung des Verlaufs der Temperatur und des Drucks in einem erfindungsgemäßen Drucktestverfahren, bei dem eine Undichtheit festgestellt wird.

### AUSFÜHRUNGSBEISPIELE

Nachstehend sind Ausführungsbeispiele der vorliegenden Erfindung anhand der beigefügten Zeichnungen beschrieben.

### Erstes Ausführungsbeispiel

### Aufbau

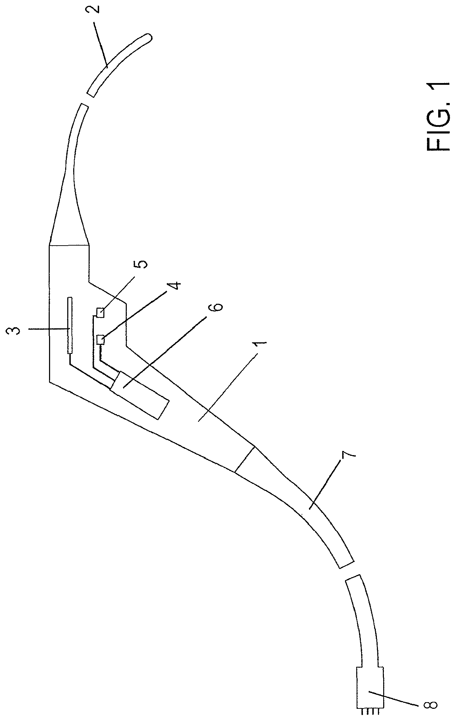

Fig. 1 zeigt ein erstes Ausführungsbeispiel der vorliegenden Erfindung in einer schematischen aufgeschnittenen Ansicht.

Ein Endoskop weist ein Endoskopgriff 1 und einen Endoskopschaft 2 auf. Im vorliegenden Ausführungsbeispiel sind der Endoskopgriff 1 und der Endoskopschaft 2 voneinander separierbar und bilden jeweils einen eigenen Innenraum aus. Am in der Zeichnung nicht dargestellten Ende des Endoskopschaftes 2, das zum Endoskopgriff 1 entgegengesetzt ist, ist z.B. eine Kamera und/oder eine LED-Beleuchtung angeordnet.

Im Innenraum des Endoskopgriffes 1 ist eine Steuereinheit 6 untergebracht, die mit einem Heizelement 3, einem Temperatursensor 4 und einem Drucksensor 5 verbunden ist und diese steuert. Die Steuereinheit 6 steuert die Betätigung der einzelnen Bestandteile des Endoskops, wie z.B. eine Kamera, eine Beleuchtungseinrichtung, ein Operationsbesteck, eine Antriebseinheit, Signalübertragungen etc. Im vorliegenden Ausführungsbeispiel steuert die Steuereinheit 6 außerdem den Betrieb des Heizelements 3, des Temperatursensors 4 und des Drucksensors 5. D.h. die Steuereinheit 6 aktiviert das Heizelement 3, so dass es sich erwärmt, und die Steuereinheit 6 empfängt Daten von dem Temperatursensor 4 und dem Drucksensor 5 und leitet diese Informationen zu einer nicht gezeigten Auswerteeinheit weiter. Die Steuereinheit 6 kann auch ein Signal über die korrekte Betätigung des Heizelementes 3 empfangen und weiterleiten, um so eine Überprüfung zu ermöglichen, dass der Betrieb des Heizelementes in der beabsichtigten Weise abläuft.

Der Endoskopgriff 1 ist mit einem Kabel 7 an der Seite des Endoskopgriffes 1, die zum Endoskopschaft 2 entgegengesetzt ist, mit einem Stecker 8 für eine Signal- und Energieübertragung versehen. Dadurch ist das Endoskop mit der Auswerteeinheit verbunden.

Das Heizelement 3 ist zumindest ein elektrischer Verbraucher (elektrischer Widerstand), der bei Endoskopbetätigung nicht involviert ist. D.h. bei der für eine Diagnose oder Behandlung erfolgenden Betätigung des Endoskops ist das Heizelement 3 nicht aktiv. Das Heizelement 3 ist derart ansteuerbar, dass es lediglich bei dem Druckdichtheitstest für das Endoskop zum Einsatz kommt und Wärme abgibt.

Der Temperatursensor 4 ist so im Innenraum des Endoskopgriffes 1 angeordnet und ausgelegt, dass er die Temperatur im Innenraum des Endoskopgriffes 1 geeignet erfassen kann. Der Temperatursensor 4 kann z.B. an einem Ort im Zentrum im Innenraum des Endoskopgriffes 1 angeordnet sein oder im oberen Bereich im Innenraum des Endoskopgriffes 1 angeordnet sein.

Der Drucksensor 5 ist so im Innenraum des Endoskopgriffes 1 angeordnet und ausgelegt, dass er den Druck im Innenraum des Endoskopgriffes 1 geeignet erfassen kann. Der Drucksensor 5 kann z.B. nahe zum Temperatursensor 4 angeordnet sein.

### Anwendung

Wenn z.B. nach einem medizinischen Einsatz und der danach erfolgten Reinigung das Endoskop einem Druckdichtheitstest unterzogen werden soll, wird über die Steuereinheit 6 das Heizelement 3 aktiviert, so dass dieses sich erwärmt. Das Heizelement 3 erwärmt die Luft im Innenraum des Endoskopgriffs 1. Der Temperatursensor 4 erfasst den Temperaturanstieg im Innenraum des Endoskopgriffs 1. Der Drucksensor 5 erfasst den Druck im Innenraum des Endoskopgriffs 1. Die Steuereinheit 6 empfängt die Daten über die Temperatur und den Druck von dem Temperatursensor 4 und dem Drucksensor 5 und leitet diese Informationen zu der Auswerteeinheit weiter. Die Auswerteeinheit zeigt dem Anwender die ermittelten Daten über die Temperatur und den Druck an.

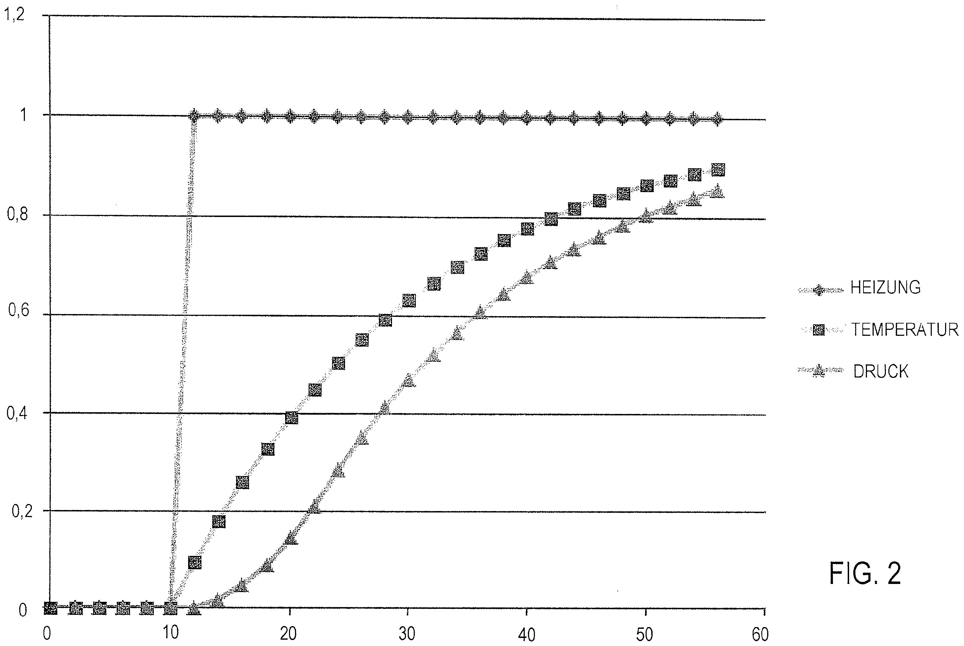

Fig. 2 zeigt eine graphische Darstellung des Verlaufs der Temperatur und des Drucks in einem erfindungsgemäßen Drucktestverfahren, bei dem keine Undichtheit festgestellt wird.

Wie in Fig. 2 gezeigt ist, wirkt das Heizelement 3 als Heizung und ist gleichmäßig aktiv (siehe die Karos "◇"). Nach Beginn des Tätigwerdens des Heizelementes 3 steigt die Temperatur zunächst annähernd gleichmäßig an, wobei sie vor Erreichen eines maximalen Endwertes nur noch geringfügig zunimmt (siehe die Quadrate "□"). Der Temperaturverlauf ist somit annähernd exponential und nähert sich dem Grenzwert der Temperatur des aktiven Heizelementes 3. Der Druck steigt (siehe die Dreiecke "Δ") bei geringer zeitlicher Verzögerung annähernd proportional zum Temperaturverlauf ebenfalls an.

Da in einem abgeschlossenen Raum gemäß der thermischen Zustandsgleichung der Druck proportional zur Temperatur zunimmt, wird bei einem Temperatur- und Druckverlauf wie in Fig. 2 geschlussfolgert, dass das geprüfte Endoskop dicht ist.

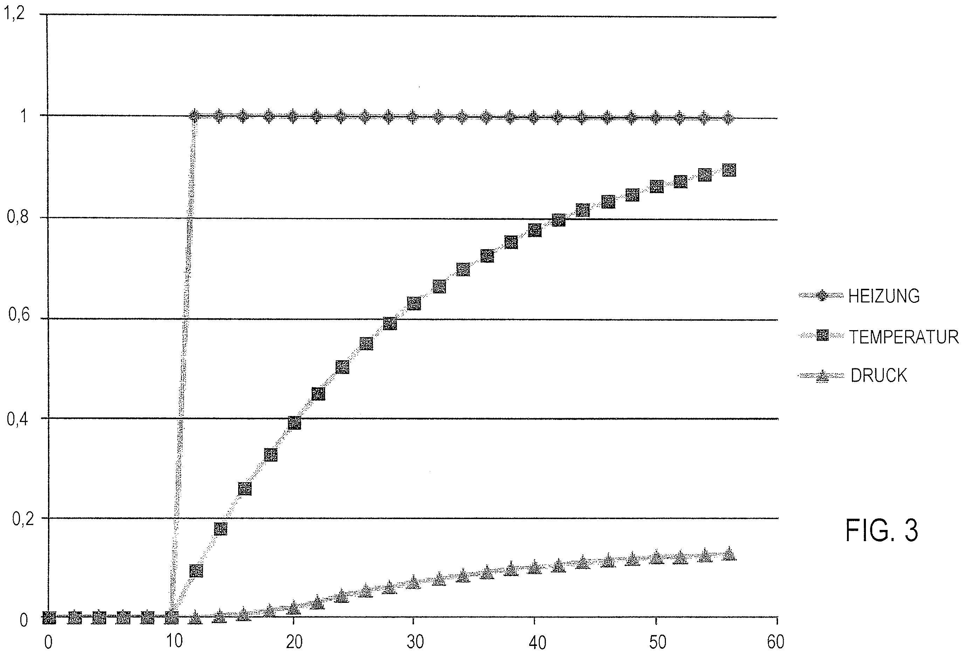

Fig. 3 zeigt eine graphische Darstellung des Verlaufs der Temperatur und des Drucks in einem erfindungsgemäßen Drucktestverfahren, bei dem eine Undichtheit festgestellt wird.

Ähnlich wie in Fig. 2 ist in Fig. 3 nach Beginn des Tätigwerdens des Heizelementes 3 der Temperaturverlauf annähernd exponential und nähert sich dem Grenzwert der Temperatur des aktiven Heizelementes 3. Der Druck nimmt jedoch nur geringfügig zu. Somit kann geschlussfolgert werden, dass Druck aus dem Innenraum des Endoskopgriffs 1 entweicht und daher das geprüfte Endoskop undicht ist.

### Zweites Ausführungsbeispiel

Im ersten Ausführungsbeispiel sind der Endoskopgriff 1 und der Endoskopschaft 2 räumlich getrennt. Im vorliegenden nicht dargestellten zweiten Ausführungsbeispiel bilden der Endoskopgriff 1 und der Endoskopschaft 2 einen gemeinsamen Endoskopinnenraum ohne eine zwischen ihnen befindliche Trennwand.

Im gemeinsamen Endoskopinnenraum sind das Heizelement 3, der Temperatursensor 4 und der Drucksensor 5 untergebracht. Dabei können das Heizelement 3, der Temperatursensor 4 und der Drucksensor 5 im Endoskopgriffabschnitt oder im Endoskopschaftabschnitt des gemeinsamen Endoskopinnenraums angeordnet sein. Das Heizelement 3, der Temperatursensor 4 und der Drucksensor 5 können auch getrennt voneinander untergebracht sein; z.B. kann das Heizelement 3 im Endoskopgriffabschnitt angeordnet sein und der Temperatursensor 4 und der Drucksensor 5 sind im Endoskopschaftabschnitt angeordnet, oder umgekehrt. Eine beliebige Anordnung ist denkbar und kann unter Berücksichtigung der spezifischen Gestaltung des Endoskops vorgenommen werden.

Die Anwendung und Funktion des zweiten Ausführungsbeispiels ist ähnlich wie im ersten Ausführungsbeispiel.

### Alternativen

Bei einigen Endoskopen kann der Endoskopgriff 1 und der Endoskopschaft 2 separat einem Druckdichtheitstest unterzogen werden. Zusätzlich zu dem Aufbau des ersten Ausführungsbeispiels können hierfür ein weiteres Heizelement 3, ein weiterer Temperatursensor 4 und ein weiterer Drucksensor 5 im Endoskopschaft 2 untergebracht sein. Dann können die Druckdichtheitstests im Endoskopgriff 1 und im Endoskopschaft 2 unabhängig voneinander durchgeführt werden. Sollte der Druckdichtheitstest das Ergebnis einer Undichtheit liefern, ist für den Anwender sofort erkennbar, ob der Endoskopgriff 1 oder der Endoskopschaft 2 undicht ist.

Das Heizelement 3 kann durch zumindest einen elektrischen Verbraucher gebildet sein, der im Endoskop vorgesehen ist und bei Endoskopbetätigung involviert ist. In diesem Fall muß kein separates Heizelement 3 im Endoskop eingebaut werden. Der zumindest eine elektrische Verbraucher kann eine elektronische Auswerteschaltung oder eine Beleuchtungs-LED sein.

Die Erfindung ist selbst bei einem Endoskop anwendbar, bei dem der Endoskopgriff 1 und/oder der Endoskopschaft 2 gegenüber der Umgebung abgedichtet und mit einem anderen Medium als Luft gefüllt sind. Vorraussetzung ist lediglich, dass dieses andere Medium wärmeleitend ist.

Um noch genauere Auswertungen der Daten über die Temperatur und den Druck von dem Temperatursensor 4 und dem Drucksensor 5 zu ermöglichen, können spezifische ideale Verlaufskurven über die Temperatur und den Druck zuvor z.B. durch Berechnungen oder Versuche erstellt und in einem dafür vorgesehenen Speicher gespeichert werden. Die spezifischen idealen Verlaufskurven betreffen dann genau das Endoskop, bei welchem der Druckdichtheitstest vorgenommen wird. Die während des Druckdichtheitstest ermittelten Daten über die Temperatur und den Druck können dann mit den gespeicherten Daten verglichen werden.

### Bezugszeichenliste

1 Endoskopgriff
2 Endoskopschaft
3 Heizelement
4 Temperatursensor
5 Drucksensor
6 Steuereinheit
7 Kabel
8 Stecker

## Patentansprüche

1. Endoskop mit
einem Endoskopgriff (1) und
einem Endoskopschaft (2),
wobei im Endoskopgriffinnenraum
ein Heizelement (3) zum Erwärmen des Endoskopgriffinnenraumes,
ein Temperatursensor (4) zum Erfassen der Temperatur des durch das Heizelement (3) erwärmten Endoskopgriffinnenraumes und
ein Drucksensor (5) zum Erfassen des Drucks des durch das Heizelement (3) erwärmten Endoskopgriffinnenraumes angeordnet sind, und
wobei der Endoskopinnenraum gegenüber der Umgebung abgedichtet ist.

2. Endoskop gemäß Anspruch 1,
wobei der Endoskopgriff (1) und der Endoskopschaft (2) einen gemeinsamen Endoskopinnenraum ausbilden.

3. Endoskop gemäß Anspruch 1,
wobei der Endoskopgriff (1) und der Endoskopschaft (2) voneinander separierbar sind und jeweils einen eigenen Innenraum ausbilden.

4. Endoskop gemäß Anspruch einem der Ansprüche 1-3,
wobei ein weiteres Heizelement (3) im Endoskopschaft (2) angeordnet ist.

5. Endoskop gemäß einem der Ansprüche 1-4,
wobei das Heizelement (3) durch zumindest einen elektrischen Verbraucher gebildet ist, der im Endoskop vorgesehen ist und bei Endoskopbetätigung involviert ist.

6. Endoskop gemäß Anspruch 5,
wobei der zumindest eine elektrische Verbraucher eine elektronische Auswerteschaltung oder eine Beleuchtungs-LED ist.

7. Endoskop gemäß Anspruch 5,
wobei der zumindest eine elektrische Verbraucher lediglich bei einer Dichtheitsprüfung aktivierbar ist.

8. Endoskop gemäß Anspruch 7,
wobei der zumindest eine elektrische Verbraucher ein separates Heizelement (3) ist, das bei Endoskopbetätigung nicht involviert ist.

9. Drucktestverfahren für ein Endoskop gemäß einem der Ansprüche 1-8 mit den folgenden Schritten:
Aufwärmen des Innenraums des Endoskops,
Erfassen des Drucks während des Aufwärmens des Innenraums des Endoskops,
Erfassen der Temperatur während des Aufwärmens des Innenraums des Endoskops und
Vergleichen des erfassten Drucks und der erfassten Temperatur.

10. Drucktestverfahren für ein Endoskop gemäß Anspruch 9,
wobei das Aufwärmen des Innenraums des Endoskops durch Aktivieren des elektrischen Verbrauchers ausgeführt wird, der im Endoskop vorgesehen ist und bei Endoskopbetätigung involviert ist.

11. Drucktestverfahren für ein Endoskop gemäß Anspruch 9,
wobei das Aufwärmen des Innenraums des Endoskops durch Aktivieren des separaten Heizelements (3) ausgeführt wird, das bei Endoskopbetätigung nicht involviert ist.

## Claims

1. Endoscope having
an endoscope handle (1) and
an endoscope shank (2),
wherein the endoscope handle interior accommodates
a heating element (3) for heating the endoscope handle interior,
a temperature sensor (4) for detecting the temperature of the endoscope handle interior heated by the heating element (3), and
a pressure sensor (5) for detecting the pressure of the endoscope handle interior heated by the heating element (3), and
wherein the endoscope interior is sealed off from the environment.

2. Endoscope according to Claim 1,
wherein the endoscope handle (1) and the endoscope shank (2) form a common endoscope interior.

3. Endoscope according to Claim 1,
wherein the endoscope handle (1) and the endoscope shank (2) are separable from each other and each form their own interior.

4. Endoscope according to one of Claims 1-3,
wherein a further heating element (3) is arranged in the endoscope shank (2).

5. Endoscope according to one of Claims 1-4,
wherein the heating element (3) is formed by at least one electrical consumer, which is provided in the endoscope and is involved in actuation of the endoscope.

6. Endoscope according to Claim 5,
wherein the at least one electrical consumer is an electronic evaluation circuit or an illuminating LED.

7. Endoscope according to Claim 5,
wherein the at least one electrical consumer is activatable only in a tightness test.

8. Endoscope according to Claim 7,
wherein the at least one electrical consumer is a separate heating element (3), which is not involved in actuation of the endoscope.

9. Pressure-testing method for an endoscope according to one of Claims 1-8, having the following steps:
heating the interior of the endoscope,
detecting the pressure during the heating of the interior of the endoscope,
detecting the temperature during the heating of the interior of the endoscope, and
comparing the detected pressure and the detected temperature.

10. Pressure-testing method for an endoscope according to Claim 9,
wherein the heating of the interior of the endoscope is effected by activating the electrical consumer which is provided in the endoscope and which is involved in actuation of the endoscope.

11. Pressure-testing method for an endoscope according to Claim 9,
wherein the heating of the interior of the endoscope is effected by activating the separate heating element (3) which is not involved in actuation of the endoscope.

## Revendications

1. Endoscope doté
d'une poignée d'endoscope (1) et
d'une tige d'endoscope (2),
dans lequel sont agencés dans l'espace intérieur de la poignée d'endoscope
un élément chauffant (3) pour réchauffer l'espace intérieur de la poignée d'endoscope,
un capteur de température (4) pour saisir la température de l'espace intérieur de la poignée d'endoscope réchauffé par l'élément chauffant (3) et
un capteur de pression (5) pour saisir la pression de l'espace intérieur de la poignée d'endoscope réchauffé par l'élément chauffant (3), et
dans lequel l'espace intérieur d'endoscope est isolé hermétiquement de l'environnement.

2. Endoscope selon la revendication 1,
dans lequel la poignée d'endoscope (1) et la tige d'endoscope (2) forment un espace intérieur d'endoscope commun.

3. Endoscope selon la revendication 1,
dans lequel la poignée d'endoscope (1) et la tige d'endoscope (2) sont séparables l'une de l'autre et forment respectivement un espace intérieur propre.

4. Endoscope selon l'une des revendications 1-3,
dans lequel un élément chauffant supplémentaire (3) est agencé dans la tige d'endoscope (2).

5. Endoscope selon l'une des revendications 1-4,
dans lequel l'élément chauffant (3) est formé par au moins un récepteur électrique, lequel est prévu dans l'endoscope et est impliqué lors de l'actionnement de l'endoscope.

6. Endoscope selon la revendication 5,
dans lequel l'au moins un récepteur électrique est un circuit électronique de contrôle ou une LED d'éclairage.

7. Endoscope selon la revendication 5,
dans lequel l'au moins un récepteur électrique est uniquement activable lors d'une vérification d'étanchéité.

8. Endoscope selon la revendication 7,
dans lequel l'au moins un récepteur électrique est un élément chauffant séparé (3) n'étant pas impliqué lors de l'actionnement de l'endoscope.

9. Procédé de test de pression pour un endoscope selon l'une des revendications 1-8 avec les étapes suivantes :
chauffage de l'espace intérieur de l'endoscope,
saisie de la pression pendant le chauffage de l'espace intérieur de l'endoscope,
saisie de la température pendant le chauffage de l'espace intérieur de l'endoscope et
comparaison de la pression saisie et de la température saisie.

10. Procédé de test de pression pour un endoscope selon la revendication 9,
dans lequel le chauffage de l'espace intérieur de l'endoscope est réalisé par activation du récepteur électrique, lequel est prévu dans l'endoscope et est impliqué lors de l'actionnement de l'endoscope.

11. Procédé de test de pression pour un endoscope selon la revendication 9,
dans lequel le chauffage de l'espace intérieur de l'endoscope est réalisé par activation de l'élément chauffant séparé (3), lequel n'est pas impliqué lors de l'actionnement de l'endoscope.
